# NEW EUROPEAN PATENT SPECIFICATION

(11) **EP 0 854 729 B2**
(45) Date of publication and mention of the opposition decision: **22.10.2008**
(45) Mention of the grant of the patent: 10.03.2004
(21) Application number: 96932269.2
(22) Date of filing: 18.09.1996
(51) Int. Cl.: A61K 39/385, A61K 39/116, A61K 39/02, A61K 39/095, C07K 1/00, A23J 1/00, C12N 1/00, A61K 39/112

(54) **IMPROVED METHODS FOR THE PRODUCTION OF NON-COVALENTLY COMPLEXED AND MULTIVALENT PROTEOSOME SUB-UNIT VACCINES**
VERBESSERTE VERFAHREN ZUR HERSTELLUNG VON NICHTKOVALENT KOMPLEXIERTEN UND MULTIVALENTEN IMPFSTOFFEN GEGEN PROTEASOMEN-UNTEREINHEITEN
PROCEDES AMELIORES DE PRODUCTION DE VACCINS POLYVALENTS A SOUS-UNITES DE PROTEOSOMES COMPLEXEES DE MANIERE NON COVALENTE

(30) Priority: 18.09.1995 US 003859 P
(43) Date of publication of application: 29.07.1998
(73) Proprietor: United States Army Medical Research Materiel Command (USAMRMC), Frederick, MD 21702-5012 (US); ID Biomedical Corporation of Quebec, Ville St. Laurent, QC H4S 2A1 (CA)
(72) Inventor: LOWELL, George, H., Cote-St.-Luc, Quebec H4W 2W8 (CA); ZOLLINGER, Wendell, D., Silver Spring, MD 20906 (US); WOOD, James, F., Germantown, MD 20874 (US)
(74) Representative: Wright, Simon Mark
(86) International application number: PCT/US1996/015002
(87) International publication number: WO 1997/010844

(56) References cited:
- EP-B1- 0 301 992
- WO-A1-94/02626
- WO-A1-94/26252
- WO-A1-95/11700
- WO-A2-90/06696
- US-B1- 4 707 543
- US-B2- 6 743 900
- DATABASE WPI Week 199522 Derwent Publications Ltd., London, GB; AN 1995-168227 XP002125408 & RU 1 522 494 C (IMMUNOPREPARAT. RES. PRODN. ASSOC. ET AL.), 15 November 1994 (1994-11-15)
- JOURNAL OF IMMUNOLOGICAL METHODS, 1990, Vol. 135, RUEGG, C.L. et al., "Preparation on Proteosome-Based Vaccines. Correlation of Immunogenicity with Physical Characteristics", pages 101-109. XP000608954
- SCIENCE, 1988, Vol. 240, LOWELL, G.H. et al., "Proteosome-Lipopeptide Vaccines: Enhancement of Immunogenicity for Malaria CS Peptides", pages 800-802. XP002055663
- ORR, N. ET AL.: 'Enhancement of Anti-Shigella lipopolysacharide (LPS) response by addition of the cholera toxin B subunit to oral and intranasal proteosome-shigella flexneri 2a LPS vaccines' INFECTION AND IMMUNITY vol. 62, no. 11, November 1994, pages 5198 - 5200, XP000608952
- LOWELL, G. H. ET AL.: 'Peptides bound to proteosomes via hydrophobic feet become highly immunogenic without adjuvants' JOURNAL OF EXPERIMENTAL MEDICINE vol. 167, February 1988, pages 658 - 663, XP002055660
- ORR, N. ET AL.: 'Immunogenicity and efficacy of oral or intranasal shigella flexneri 2a and shigella sonnei proteosome lipopolysacharide vaccines in animal models' INFECTION AND IMMUNITY vol. 61, no. 6, June 1993, pages 2390 - 2395, XP002958580
- LOWELL, G. H. ET AL.: 'Intranasal and intramuscular proteosome staphylococcal and efficacy against lethal SEB intoxication in mice' INFECTION AND IMMUNITY vol. 64, no. 5, May 1996, pages 1706 - 1713
- LIVINGSTON, P.O. ET AL.: 'GD3/ Proteosome vaccines induce consistent IgM antibodies against the ganglioside GD3' VACCINE vol. 11, no. 12, 1993, pages 1199 - 1204, XP001088998
- Proc. Natl. Acad. Sci. USA, 1995, Immunology, WESTERINK, A. J. et al Vol. 92, pp.4021-4025
- The Journal of Clin. Invest. 1979, ZOLLINGER, W. D. et al., Vol. 63; p. 836-848
- Vaccine, 1995. BOSLEGO, J. et al., Vol. 13, p. 821-829 (Published June 1995)
- Infection and Immunity, 1984. ZOLLINGER, W. D. et al., Vol. 46, No. 1, p. 260-266.
- NIPH ANNALS. SIERRA, GVG et al., 1991. Vol. 14, p. 195-207.
- NIPH ANNALS. FREDRIKSEN et al., 1991. Vol. 14, p. 67-79.
- Vaccine. VAN DER LEY, P. et al., 1995. Vol. 13, p. 401-407.
- Infection and Immunity. VAN DER LEY, P. and POOLMAN, J. T., 1992. Vol. 60, p. 3156-3161.
- Infection and Immunity. VAN DER LEY, P. et al., 1993. Vol. 61, p. 4217-4224.

## Description

### 1. FIELD OF THE INVENTION

This invention concerns methods of production and compositions for non-covalently complexed multivalent proteosome vaccines for mucosal and parenteral administration.

### 2. BACKGROUND OF THE INVENTION

In order for multivalent sub-unit vaccines to stimulate optimal immune responses to each of the components, the proper components should be appropriately associated and each be available to the immune system so that they may be efficiently recognized and processed by cells of the immune system. Prime examples of such non-covalently complexed vaccines include proteosome vaccines which can consist of neisserial outer membrane protein proteosomes non-covalently complexed to a wide variety of antigens including peptides, lipopeptides, transmembrane or toxoided proteins, polysaccharides or lipopolysaccharides (LPS) (patent application nos. 07/065,440 filed 23 June 1987 "Immunogenic peptide vaccines and methods of preparation"; 07/336,952 filed 12 April 1989 Immunopotentiaing system for large proteins and polypeptides"; 07-958,426 filed 8 October 1992 "Oral or Intranasal Vaccines Using Hydrophobic Complexes Having Proteosomes and Lipopolysaccharides"; 08/029,666 filed 11 March 1993 "Immunopotentiating Systems for Preparation of Immunogenic Materials"; 08/143,365 filed 29 October 1993 "Immunopotentiating Systems for Preparation of Immunogenic Materials"; 93/10,402 filed 29 October 1993 "Submicron Emulsions as Vaccine Adjuvants"; 08/063,613 filed 18 May 1994 Solid Fat Nanoemulsions as Vehicles for Vaccine Delivery" and publications Orr, N., Robin, G., Cohen, D., Arnon, R. and Lowell, G.H. (1993). Immunogenicity and Efficacy of Oral or Intranasal Shigella flexneri 2a and Shigella sonnei Proteosome-Lipopolysaccharide Vaccines in Animal Models. Infect. Immun. 61:2390; Mallett, C.P., T.L. Hale, R. Kaminski, T. Larsen, N. Orr, D. Cohen, and G.H. Lowell. 1995. Intranasal or intragastric immunization with proteosome-Shigella lipopolysaccharide vaccines protect against lethal pneumonia in a murine model of shigellosis. Infect. Immun. 63:2382-2386.; Lowell GH, Kaminski RW, Grate S et al. (1996) Intranasal and intramuscular proteosome-staphylococcal enterotoxin B (SEB) toxoid vaccines: immunogenicity and efficacy against lethal SEB intoxication in mice. Infec. Immun. 64:1706-1713.; Lowell, G. H. (1990) Proteosomes, Hydrophobic Anchors, Iscoms and Liposomes for Improved Presentation of Peptide and Protein Vaccines. in New Generation Vaccines: G. C. Woodrow and M. M. Levine, eds. (Marcel Dekker, NY). Chapter 12 (pp. 141-160) and Lowell, G.H., W.R. Ballou, L.F. Smith, R.A. Wirtz, W.D. Zollinger and W.T. Hockmeyer. 1988. Proteosome-lipopeptide vaccines: enhancement of immunogenicity for malaria CS peptides. Science 240:800.

Ruegg C.L. et al., 1990 (Journal of Immunological Methods, Vol. 135: 101-109) disclose the preparation of proteosome-based vaccines. Lowell G.H. et al., 1988 (Science 240: 800-802) disclose proteosome-lipopeptide vaccines and enhancement of immunogenicity for malaria CS peptides. Lowell G.H. et al., 1988 (Journal of Experimental Medicine, Vol. 167: 658-663) disclose that peptides bound to proteosomes via hydrophobic feet become highly immunogenic without adjuvants.

For practical application in administering vaccines to protect against disease, it is frequently necessary to deliver several such antigens at the same time usually due to the fact that individuals are susceptible to the contraction of diseases caused by a variety of organisms. Moreover, several organisms, whether or not they are related to one another, often are endemic in the same location and therefore individuals requiring protection may need vaccination with several types of vaccines.

In the past, the production of vaccines that require non-covalent complexing of components has been accomplished using simple dialysis in which components are placed in dialysis tubing in the presence of dialyzable detergent and the mixture is dialyzed for 7-10 days to attempt to remove the detergent. The practical disadvantages of this system tend to severely preclude the advanced development and commercialization of this technology for several reasons including 1) Time: Length of time of the procedure: The need to use GMP resources for weeks while the vaccine is dialyzing is impractical both due to the excess costs involved and the increased opportunity for breakdown or contamination of mechanical or biological components during this extended period of time; 2) Contamination: Increased opportunity for contamination: dialysis tubing is difficult to sterilize, dialysis tubing requires manually opening and closing the system thereby exposing the components to contamination during both the loading and unloading process. Since many days transpire between loading and unloading the tubing, the risk of a small contamination in the initial days of the process may readily be magnified during the many days of dialysis to render this method useless for practical vaccine manufacture. The risk of puncturing the bag can result in loss of product. 3) Temperature: Necessity to perform the dialysis at 4° C due to the extensive time involved; 4) Volume of dialyzing fluids: In order to manufacture vaccine for scale-up of the process, the use of massive amounts of dialysis fluid would be necessary since a 200: ratio of liquid outside to the dialysis tubing to inside the tubing is typically required. Therefore, for example, the production of a pilot lot of two liters of vaccine would require 400 liters of fluid outside the tubing per day - 4,000 liters per 10 days - and the production of a production lot of 20-200 liters would require 40,000-4,000,000 liters. These amounts are wasteful and impractical compared to the method used in the instant invention; Dialysis tubing is not scalable since large amounts of product is problematic and 5) Inability to readily measure completeness of removal of the detergent so as to maximize vaccine effectiveness. Since the dialysis bag is placed in a container with 200 volumes of buffer, the ongoing measurement of detergent removal is neither practical nor feasible and 6) In addition, no method has been described for the measurement of the presence of the detergent used in the preferred embodiment, Empigen BB.

The second problem solved in this invention is the demonstration of the method of producing and delivering multivalent vaccines. Components can either be made together or produced separately and mixed together prior to administration. The instant invention demonstrates the optimal way of preparing such multivalent vaccines.

### 3. SUMMARY OF THE INVENTION.

The subject of the instant invention broadly relates to the production and manufacture of proteosome-amphiphilic determinant vaccines designed for either parenteral or especially for mucosal administration including, but not limited to, respiratory (e.g. including intranasal, intrapharyngeaeal and intrapulmonary), gastro-intestinal (e.g. including oral or rectal) or topical (e.g. conjunctival or otic) administration to induce both systemic (serum) and mucosal (including respiratory and intestinal) antibody responses. An amphiphilic determinant is a molecule having hydrophobic and hydrophilic regions which, when appropriately formulated with proteosomes, align with the proteosomes to form a complex which elicits an immunologic response in a subject. Typical amphiphilic determinants include glycolipids, liposaccharides (including detoxified lipopolysaccharides), lipopeptides, transmembrane, envelope or toxoided proteins, or proteins or peptides with intrinsic hydrophobic amino acid anchors. These determinant materials can be obtained from gram negative bacteria including *escherichia, klebsiella, pseudomonas, hemophilus brucella, shigella and neisseria.* More specifically, the invention relates to proteosome vaccines in which meningococcal outer membrane protein proteosome preparations (prepared from any strain of *N. meningiditis* or *N. gonorrhea* or other neisserial species) are non-covalently complexed to native or detoxified shigella or neisserial lipopolysaccharides or lipooligosaccharides to form vaccines designed to protect against diseases caused by gram negative organisms that contain any of the component parts of the complex including meningococci or shigellae. More specifically, the invention relates to proteosome vaccines that contain LPS that induce antibody responses that recognize type-specific somatic polysaccharide O-antigens of shigella lipopolysaccharides and thereby confer homologous protection against shigellosis. Still more specifically, the lipopolysaccharides that, when complexed to proteosomes induce such anti-shigella protective immune responses are prepared and purified from either *Shigella sonnei* or *Plesiomonas shigelloides* for immunity against *Shigella sonnei* disease, from *Shigella flexneri 2a* for immunity to *Shigella flexneri 2a* disease, and so forth, using LPS derived from homologous or antigenically cross-reacting organisms to confer homologous immunity against shigellosis caused by *S*. *flexneri 2a* (or *3a* etc.), *S. boydii, S. sonnei* etc. Still more specifically, the instant invention describes the successful administration of proteosome-shigella vaccines that are multivalent in that two independently made proteosome vaccines using shigella LPSs derived from *S. flexneri 2a* (for *S. flexneri 2a* disease) and from *P. shigelloides* or *S. sonnei* (for S. sonnei disease) are administered together thereby inducing antibodies that recognize the two organisms and thereby conferring protection against the two types of diseases. Most specifically, the instant invention relates to a proteosome-shigella LPS vaccine in which proteosomes from group B type 2b meningococci are complexed to *P. shigelloides* LPS using hollow fiber diafiltration technology to produce a vaccine administered by mucosal respiratory and/or gastro-intestinal routes to induce antibodies that recognize the somatic O-antigen LPS of *S. sonnei* and thereby protect against shigellosis caused by this organism. Other conventional ultraftltration/diafiltration are envisioned, e.g. platform membrane and membrane cartridge.

The present invention provides methodology to produce non-covalently complexed vaccines in a manner that 1) Decreases the time required, 2) Decreases the opportunity for contamination, 3) Increases the temperature to ambient temperature that such vaccines can be produced, 4) Allows for efficient scale-up of the production process so as to require minimum use of reagents, 5) Allows for reliable and efficient sampling of dialysate so as to be able to repeatedly measure rate of removal of the detergent so as to optimize efficiency of the operation. This leads directly to an increase in the complexing efficiency of vaccine so as to produce vaccine with measurably greater immunogenicity at lower doses. In this manner, the overall quality of the product is significantly enhanced. 6) In addition, a method is described to measure the presence of the detergent used in the preferred embodiment, Empigen BB. Other dialyzable detergents can be used in place of Empigen BB. Furthermore, using the method of the instant invention, it has been demonstrated that the vaccine can be lyophilized and re-hydrated in such a manner as to retain optimal vaccine potency as measured by vaccine immunogenicity.

The method of the instant invention entails the use of a hollow fiber ultrafiltration / diafiltration cartridge to effect complexing by removal of the detergent. By varying the size of the housing of the cartridge, the time of dialysis for production of a lot of vaccine can be reduced from > 7-10 days to less than 72 hours and usually less than 48 or 24 hours. Since this short time is used, the reaction temperature can be increased from 4° to normal room temperature without compromising the process or the integrity of the products. While the process was performed at about 20°C, temperatures between 0° and 40°C are contemplated. Since the system is closed, there is a highly reduced potential for contamination. In addition, by increasing the size of the housing, an exceedingly large amount of material can be processed in a relatively short period of time thereby allowing for efficient and reproducible scale-up of the procedure for commercial development. Furthermore, the permeate can be repeatedly sampled to measure the removal of the detergent as can be accomplished using the test of the instant invention to measure the presence of the detergent empigen BB, the detergent used in the preferred embodiment. The uniqueness of this methodology needed experimental verification since it was not obvious that the complexing and the structure of the vaccine into immunogenic materials that was accomplished by slowly dialyzing over 7-10 days using a stationary dialysis bag could be equalled or improved upon using the hollow fiber technology in which the moieties to be complexed are moving through tubes at exceedingly high flow rates compared to that of the stationary dialysis tube.

Since the nominal molecular weight cutoff of the membrane used can be selected to be from 1,000, 3,000, 5,000, 10,000, 30,000, 50,000 or greater, depending on the size of the components to be non-covalently complexed, this system is readily adaptable to the complexing of native or detoxified lipopolysaccharides, lipids, peptides, lipopeptides, liposaccharides, polysaccharides, gangliosides or transmembrane, envelope or native or toxoided proteins to each other or to meningococcal outer membrane protein preparations of proteosomes.

The resultant product can be used as a vaccine administered either parenterally or mucosally i.e. via the respiratory or gastro-intestinal tract e.g. intranasally, orally, by oropharyngeal inhalant, topically or rectally. In the example given, it is shown that proreosomes are non-covalently complexed to *P. shigelloides* LPS to form a vaccine that induces the anti-*S. sonnei* LPS responses necessary for protection against *S. sonnei* shigellosis.

The methodology has three stages: a preliminary operation; complexing the proteosome with an amphiphilic determinant e.g. *P. Shigelloides* LPS; followed by a sterile filtration.

To optimally deliver multivalent vaccines, the present invention shows that the best method is to make the specific vaccines individually and then immunize with the two individually made vaccines, each at the optimal concentration, on the same day. Other possibilities such as making hybrid vaccines during the formation of the non-covalent complexes have also been accomplished or are envisioned.

### 4. BRIEF DESCRIPTION OF THE FIGURES

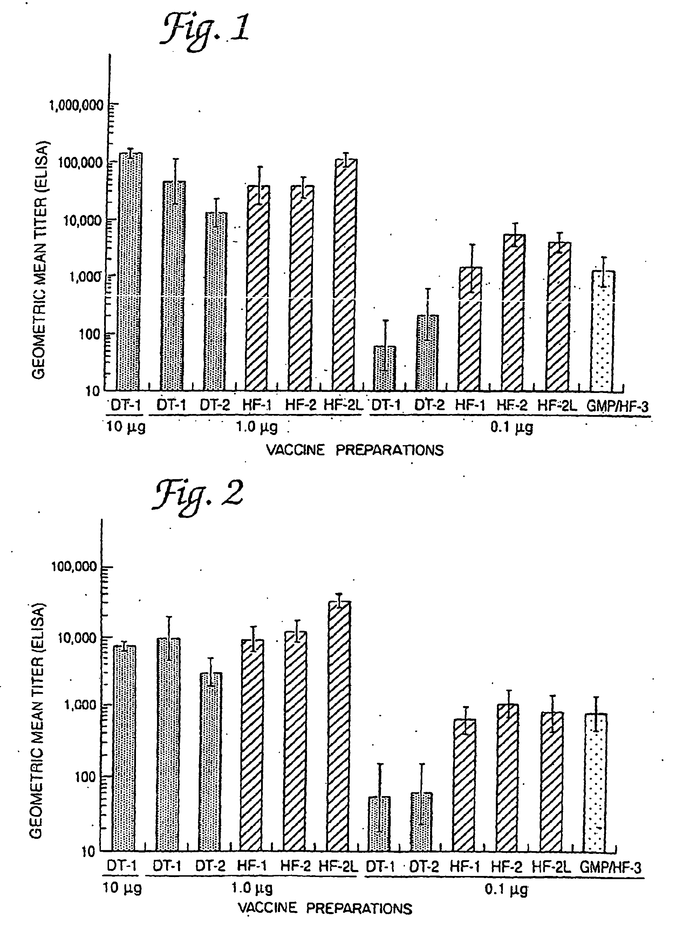

- Figure 1:: Immunogenicity of several preparations of the proteosome-*Plesiomonas shigelloides* LPS vaccine: anti-*Shigella sonnei* LPS IgG titers in murine sera after two intranasal immunizations of vaccine containing 10, 1.0, or 0.1 µg of Protein. **DT-1 & DT-2** - dialysis tubing. **HF-1 & HF-2**- hollow fiber cartridge. **HF-2L** - hollow fiber cartridge and lyophilized. **GMP** - good manufacturing procedures.
- Figure 2:: Immunogenicity of several preparations of the proteosome-*Plesiomonas shigelloides* LPS vaccine: anti-*Shigella sonnei* LPS IgA titers in murine sera after two intranasal immunizations of vaccine containing 10,1.0, or 0.1 µg of Protein.

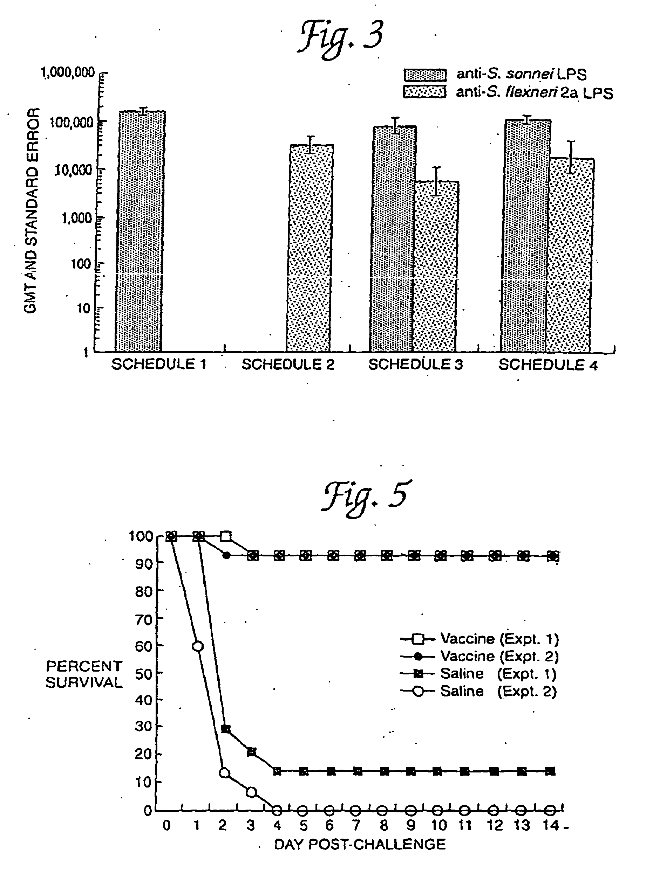

- Figure 3:: Mouse serum IgG titers following intranasal immunization with the proteosome-*Shigella* LPS vaccines: simultaneous and/or sequential administration of vaccines for *S. sonnei* and *S. flexneri 2a*. **Schedule 1** consisted of five mice intranasally immunized on days 0 and 21 with the proteosome-*S. sonnei* LPS vaccine (10 micrograms each of proteosomes and LPS). Mice were bled four weeks after the last immunization. **Schedule 2** consisted of five mice intranasally immunized on days 0 and 21 with the proteosame-*S*. *flexneri 2a* LPS vaccine (10 micrograms each ofproteosomes and LPS). Mice were bled four weeks after the last immunization. **Schedule 3** consisted of five mice intranasally immunized on days 0 and 21 with the proteosome-*S*. *sonnei* LPS vaccine (10 micrograms each of proteosomes and LPS) and the proteosome-*S. flexneri 2a* LPS vaccine (10 micrograms each of proteosomes and LPS). Mice were bled four weeks after the last immunization. **Schedule 4** consisted of four mice intranasally immunized on days 0 and 21 with the proteosome-*S. sonnei* LPS vaccine (10 micrograms each of proteosomes and LPS) and then on days 49 and 70 with the *S. flexneri 2a* LPS vaccine (10 micrograms each of proteosomes and LPS). Mice were bled four weeks after the last *S. flexneri 2a* immunization. Administration of the proteosome-*P. shigelloides* LPS (for *S. sonnei* shigellosis) and the proteosome-*S. flexneri 2a* vaccines together on the same day or weeks apart results in good immunogenicity to both the *S. sonnei* and *S. flexneri 2a* LPS components.

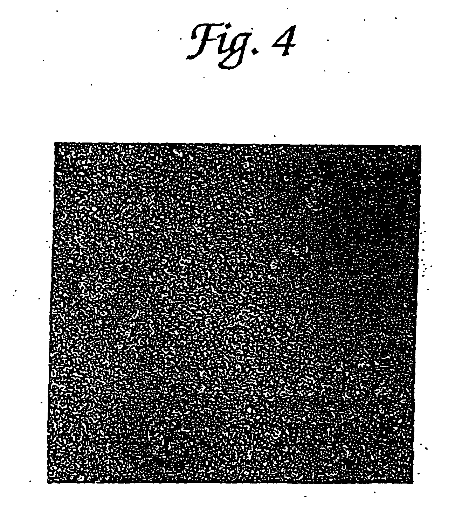

- Figure 4:: Electron micrograph of proteosome-*P. shigelloides* LPS vaccine showing gold-labelled anti-*shigella sonnei* LPS associated with proteosomes. In this micrograph, gold-labelled secondary antibodies (visible as opaque black dots) identify primary antibodies directed against shigella LPS. As can be seen, the LPS is clearly associated with the proteosome vesicles.
- Figure 5:: Proteosome-P. Shigelloides LPS vaccine protects against lethal *shigella sonnei pneumonia* in an animal model of *Shigellosis.* Groups of outbred mice (14 to 15 per group) were immunized intranasally on weeks zero and three with the proteosome-*P. Shigelloides* LPS vaccine (10 micrograms each of proteosomes and LPS per 20 microliters). Control groups were given saline on the same schedule. All mice were challenged intranasally with five to ten million colony forming units of *S. sonnei* on week seven. The mice were observed daily for two weeks after challenge, and mortality resulting from the ensuing pulmonary disease was recorded. As we have shown previously, 86 to 100% of the control mice die within four days after challenge. In both experiments, 93% of mice immunized with the vaccine survived challenge (*P* < 0.001, Fisher's Exact Test).

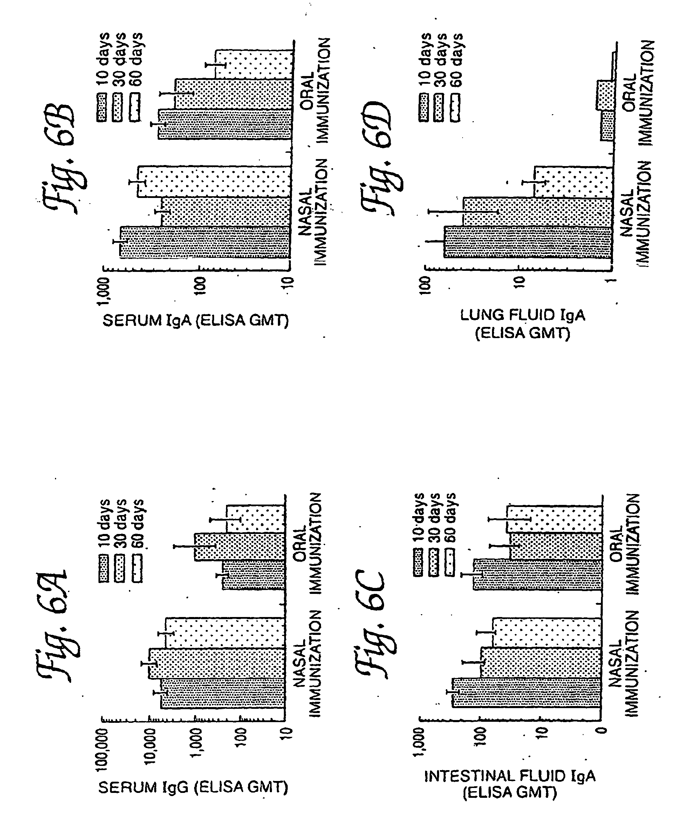

- Figure 6:: Intranasal or oral immunization with proteosome-shigella flexneri 2a LPS vaccines induce anti-shigella LPS IgG and IgA in serum and IgA in intestinal and lung lavage fluids that can last 30-60 days post-immunization.

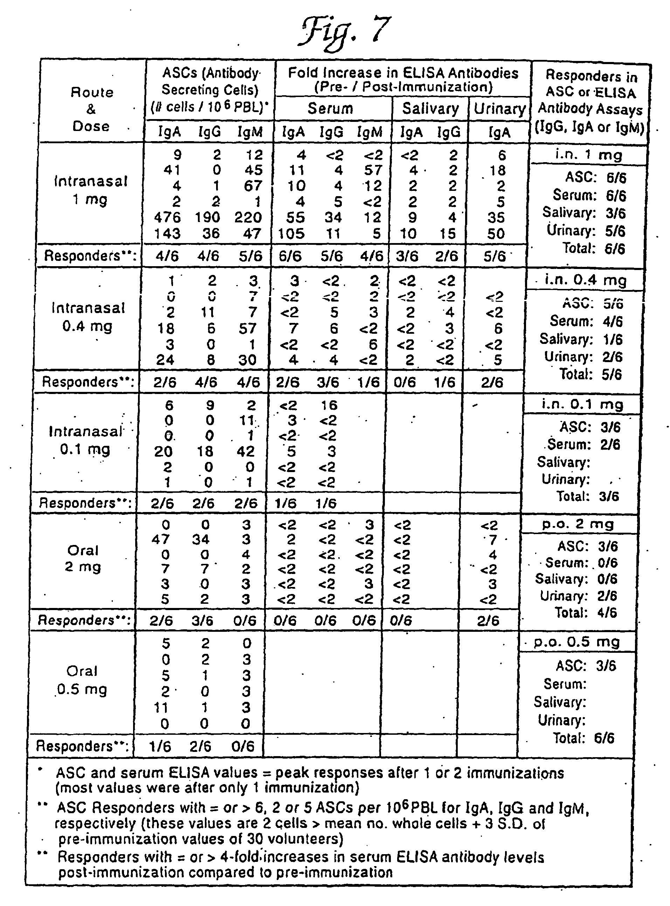

- Figure 7:: Intranasal or oral immunization of humans with one or two doses proteosome-*p. shigelloides* LPS vaccines for *Shigella sonnei* using different vaccine amounts: Induction of anti-shigella LPS IgA, IgG and IgM peripheral blood ASC responses, and serum, salivary and urinary antibody responses.

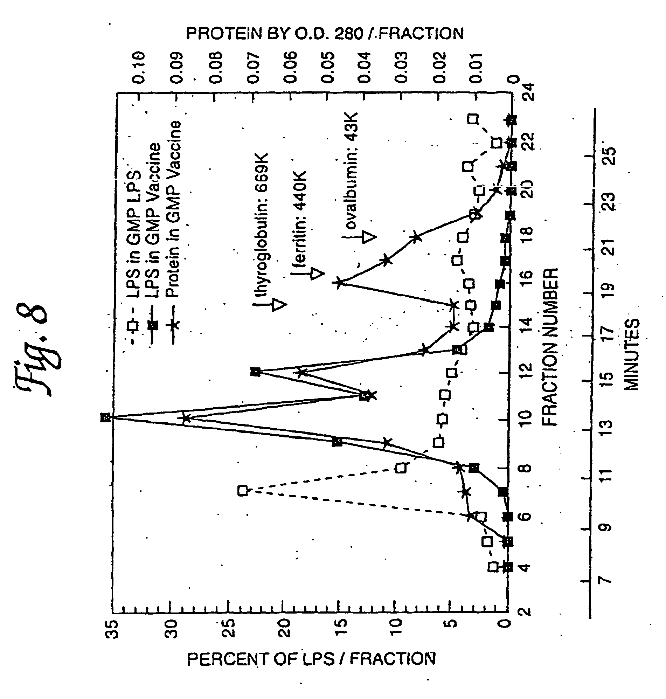

- Figure 8:: LPS in HPLC fractions after applying uncomplexed *P*. *shigelloides* LPS and Demonstration of non-covalent complexing of LPS and proteosome proteins by co-elution of LPS and proteins in HPLC fractions after applying proteosome-*P. shigelloides* LPS vaccine.

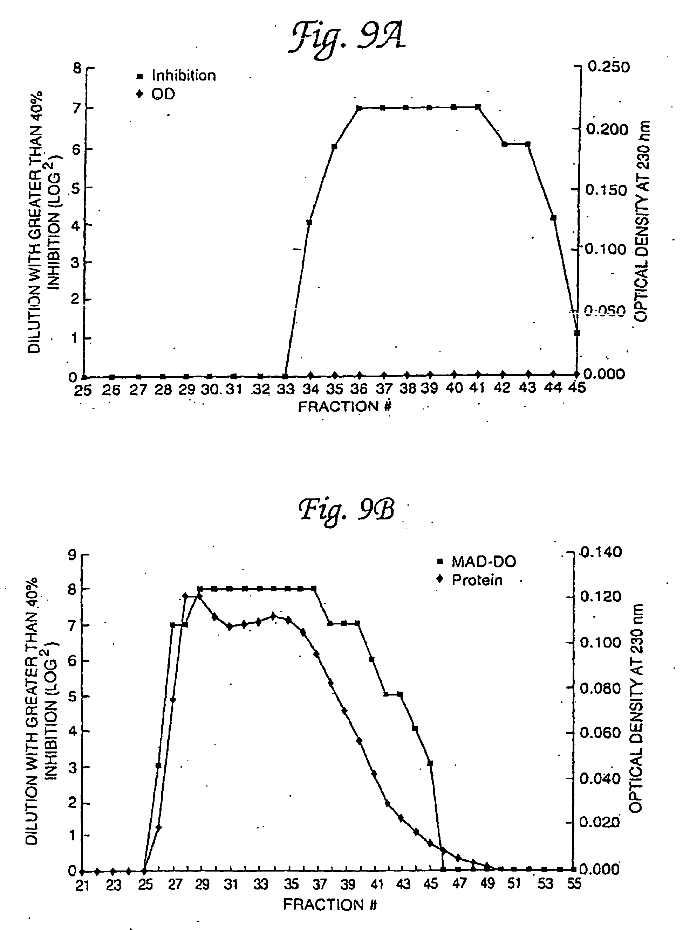

- Figure 9:: Bactericidal antibody response of mice to meningococcal outer membrane protein-detoxified lipooligosaccharide vaccine, geometric mean reciprocal titers against meningococcal strain 9162

### 5. DETAILED DESCRIPTION OF THE INVENTION:

### EXAMPLE 1

This example is detailed for production of proteosome-*P. shigelloides* vaccine containing approximately 2-3 grams of protein. The procedure is equally applicable for scale-up using 10-1000 fold more material with appropriate scale-up of housing size of the membrane cartridge and appropriate increases in the volumes. Using the appropriate size membranes with appropriate size molecular weight cutoffs to retain the antigens to be complexed, this procedure is equally applicable for complexing of proteosomes to other native or detoxified lipopolysaccharides, lipids, peptides, lipopeptides, liposaccharides, polysaccharides, gangliosides or transmembrane, envelope or native or toxoided proteins to each other or to meningococcal outer membrane protein preparations of proteosomes. Empigen BB is the detergent used as an example in this procedure; the procedure applies equally to any dialyzable detergent that solubilizes the components. This procedure uses A/G technology cartridges but is equally applicable to any brand cartridge type ultrafiltration/diafiltration system.

### 5.0.0.1 PRELIMINARY OPERATION

5.0.0.1. Sterile filter a stock solution of 30% Empigen BB, which is the detergent used in this process.
5.0.0.2. Prepare 2X TEEN / 2% Empigen (0.1M Tris, 0.02 M disodium EDTA, 0.3 M sodium chloride, WFI and 2% Empigen, pH 8.0)
5.0.0.3. Prepare 150 L of a TNS (Tris Normal Saline) solution containing 0.05M Tris, 0.15M sodium chloride pH 8.0 for the purpose of dialyzing out the detergent.
5.0.0.4. Prepare 10 liters of Sodium Hydroxide for UF cartridge cleaning.
5.0.0.5. Thaw the required amount of *Shigella flexneri 2a* Lipopolysaccharide (LPS).

### 5.0.1. COMPLEXING PROTEOSOMES WITH S. flexneri 2a LPS

5.0.1.6. Add an equal volume of 2X TEEN buffer to the volume of LPS and mix well.
5.0.1.7. Thaw and measure an amount of bulk proteosomes, in mg's, equal to the amount of LPS thawed in step 5.
5.0.1.8. To the proteosomes, add 30ml of sterile filtered empigen per liter. Mix well and combine with the LPS from step 5.
5.0.1.9. Mix both components well. Usually 15 minutes on a stir plate and stir bar combination is adequate.
5.0.1.10. To dialyze the detergent out so that proteosome complexes could form, set up an ultrafiltration system (based on tangential flow technology} using a hollow fiber cartridge with a nominal molecular weight cutoff (NMWC) pore size of 10,000 manufactured by A/G Technology, Inc. connected to sterilized silicone tubing and a peristaltic pump. Sanitary pressure gauges are placed on the inlet and outlet side of the cartridge to monitor pressure throughout the run. A back pressure valve is placed on the outlet side of the cartridge to as to adjust back pressure.
5.0.1.11. The ultrafiltration system is cleaned and sanitized with WFI and 0.5N NaOH at a temperature of 50°C for a time greater than 60 minutes. This system is flushed with WFI and equilibrated with TNS buffer prior to use.
5.0.1.12. A sterile reservoir vessel is placed in line so that the inlet liquid is pulled from the vessel and the return retentate returned to the same reservoir. As liquid passes through the cartridge membrane (permeate or filtrate) it is replenished by TNS buffer either by direct addition or by a continuous feed system. Set up a continuous feed system by connecting silicone tubing to a vessel containing TNS buffer to the sample reservoir that is then air tight. As the liquid is removed by filtration from the sample reservoir a vacuum is created causing "TNS" buffer to be pulled from the its vessel into the sample reservoir. If the system remains air tight the sample level it the reservoir remains constant.
5.0.1.13. Start the recirculation pump to begin diafiltration. Adjust the pump speed and the back pressure valve to obtain a back pressure of 15 ±4 psi.
5.0.1.14. Verify progressive removal of Empigen BB by testing permeate samples taken every 10-15 liters using the Empigen Precipitin Test. This test consists of making serial dilutions of the permeate and of a standard solution containing a known quantity of empigen BB, adding HCl to acidify the solutions and then adding serial dilutions of SDS (sodium dodecyl sulfate) to form a checkerboard-type assay. Maximum precipitate will form when equivalent amounts of empigen and SDS are present. In this manner, the amount of empigen present can be quantitated by noting the dilution of the SDS, the permeates and of the standards at which the maximum precipitate is formed. As the diafiltration progresses, the amount of empigen becomes less and the tube containing less SDS will be the tube with the maximum precipitate. The presence of precipitate is quantitated by measuring the OD at 600 nm using a spectrophotometer.
5.0.1.15. For the amount of components used in this example, continue diafiltration until at least 120 liters of permeate has been processed and there is a lack of significant precipitate in the Empigen Precipitin Test (Maximum OD 600nm is less than 0.05).
5.0.1.16. After dialysis is complete concentrate the product to a final volume to get an OD₂₈₀ value close to 6.0. Drain the system and wash with 300-400ml of "TNS" buffer. Drain the system again and combine both pools. Clean the cartridge by recirculating large amounts of WFI followed by 0.5N NaOH at 50°C for >60 minutes. Rinse out the NaOH with WFI and store the system in 0.01N NaOH.

### 5.0.2. STERILE FILTRATION

5.0.2.17. The complexes may, if desired, be sterile filtered. Proteosome concentration can be adjusted before or after 0.22µ filtering. Sterile filtration is normally performed with filter units from Millipore Corp. These units are called Millipaks and they come in various sizes. The filtered bulk is stored at 4°C until the fill operation and is tested for sterility.
5.0.3.0 **SPECIFIC PURPOSE:** Combine bulk GMP preparations of meningococcal outer membrane protein proteosomes and *S. flexneri 2a* lipopolysaccharide into non-covalent complexes by removal of detergent.
5.0.3.1 **APPLICATIONS:** This GMP procedure results in the production of a bulk preparation formation of non-covalent complexes of proteosomes with *S. flexneri 2a* lipopolysaccharide for use as a vaccine against *S. flexneri 2a* infection.

### EXAMPLE 2

Noncovalent complexing of bulk *Neisseria meningitidis* strain 9162 purified outer membrane proteins (proteosomes) and alkaline detoxified *N. meningitidis* L8 lipooligosaccharide purified from strain 8532.
5.1.0 Bulk 9162 outer membrane protein (proteosomes), lot 0136 and bulk detoxified meningococcal L8 Lipooligosaccharide (LOS) lot 0203 were taken from storage and thawed at room temperature. A volume (182 ml) of the bulk LOS containing 500 mg LOS in sterile distilled water was combined with a volume (306 ml) of bulk proteosomes containing 400 mg of protein in buffer containing 0.05 M Tris-HCl, 0.15 M NaCl, 0.01 M EDTA, and 0.1 % Empigen BB.
5.1.2 Empigen BB (30% solution) was sterile filtered through a 0.22 µm pore size filter and 1/60^{th} volume added to the combined proteosomes, LOS solution and stirred at room temperature for 1 hour.
5.1.3 The detergent buffer solution was removed from the proteosomes, LOS solution and replaced with sterile distilled water by ultrafiltration using an A/G Technology ultrafiltration cartridge UFP-3-C-6 with a 3000 molecular weight cut off pore size.
5.1.4 The procedure for set up, sanitization, washing, washing, and cleaning of the apparatus was the same as described in example 1. Inlet pressure was 15 ± 4 PSI and the permeate flow rate was 175 ml per minute.
5.1.5 The permeate was tested after each 3 to 4 liters for the presence of Empigen BB detergent by the precipitation method described in example 1. The ultrafiltration was continued until no precipitate was obtained in the test plus an additional 5 liters. A total of 37 liters of permeate was collected.
5.1.6 The retentate was clear and was sterile filtered through a 0.22 µm pore size filter assayed for protein and LOS and stored as a bulk product at 4°C.
5.1.7 The bulk product was diluted to a concentration of 0.2 mg of protein per ml and the concentration of NaCl adjusted to 0.15 M. Thimerasol at 0.01 % was added as a preservative, and the resulting bulk was dispensed under sterile conditions into final container vials, labelled and stored at -70°C.
5.1.8 This product was tested in mice for immunogenicity when given as a saline solution and adsorbed to aluminum hydroxide gel as adjuvant. The results are given in the Table 1 below and show that the vaccine complexes were immunogenic when given i.p. in mice.

**Table 1.**

| Bactericidal antibody response of mice to meningococcal outer membrane protein-detoxified lipooligosaccharide vaccine, geometric mean reciprocal titers against meningococcal strain 9162 | | | | | |
|---|---|---|---|---|---|
| Vaccine | Dose | Adjuvant | Day 0 | Day 28 | Day 42 |
| proteosomes-dLOS Lot 0271 | 1 µg | None | < 8 | < 8 | 16 |
| | 3 µg | None | <8 | <8 | 128 |
| | 10 µg | None | <8 | <8 | 512 |
| | | | | | |
| proteosomes-dLOS Lot 0271 | 1 µg | Al(OH)₃ | <8 | <8 | 512 |
| | 3 µg | Al(OH)₃ | <8 | <8 | 512 |
| | 10 µg | Al(OH)₃ | < 8 | 64 | 2048 |
| Note: Vaccine was given intraperitoneally at 0 and 28 days to groups of ten outbred CD-1 mice. | | | | | |

5.1.9 The complexing of the proteosomes and LOS was verified by column chromatography of the proteosomes and LOS components before and after complexing (Figure 9). Chromatographic analysis of bulk meningococcal L8 LOS and the final proteosomes/LOS complexes on Sephacryl S300 low pressure column. The column was run in 0.05 M Tris-HCl, 0.01 M EDTA, 0.15 M NaCl buffer. The assay for protein was by absorbance at 280 nm, and the assay for LOS was by inhibition of an ELISA (enzyme linked immunosorbant assay) in which an L8 monoclonal antibody binding to purified L8 LOS adsorbed to the plastic plate was inhibited by serial dilutions of the fractions off the Sephacryl column. Before complexing, the LOS came off the column in a broad peak centered at fraction 39. After complexing, the LOS co-eluted with the proteosomes in a peak approximately centered at fraction 33.
5.1.10 **SPECIFIC PURPOSE:** Combine bulk GMP preparations of meningococcal outer membrane protein proteosomes and detoxified meningococcal lipopolysaccharide into non-covalent complexes by removal of detergent.
5.1.11 **APPLICATIONS:** This GMP procedure results in the production of a bulk preparation formation of non-covalent complexes of proteosomes with detoxified meningococcal lipopolysaccharide for use as a vaccine against *N. meningitides* (meningococcal) infection.

### EXAMPLE 3

This example is also directed to the production of proteosome-*P. shigelloides* vaccine. The procedure is equally applicable for scale-up using 10-1000 fold more material with appropriate scale-up of housing size of the membrane cartridge and appropriate increases in the volumes.
5.8.1 Preparation of 25 mL Sterile-Filtered Empigen BB (30% Solution)
   5.8.1.1 Use a 100 ml Nalgene disposable filter unit with 0.2 µ pore size membrane filter to sterile filter about 25 ml of Empigen BB (30% solution).
5.8.2 Prepare 4 L of TEEN 2x with 2 % Empigen BB (consisting of 0.1 M Tris, 0.02 M disodium EDTA, 0.3 M sodium chloride, WFI and 2% solution of empigen BB).
5.8.3 Prepare a 10 L solution of "TNS" fifteen times to total 150 liters: TNS consists of 0.15 M sodium chloride, 0.05 M Tris Buffer pH 8.0 ± 0.2
5.8.4 Prepare 10 liters of 0.5 N Sodium Hydroxide
5.8.6 If necessary, thaw bulk *P. shigelloides* lipopolysaccharide (LPS) to prepare for complexing with proteosomes and record the lot number, and concentration of the LPS, the required amount of LPS in mg and the calculated volume of LPS to remove.

### 5.9.0 COMPLEXING PROTEOSOMES with P. shigelloides LPS

### 5.9.1 PREPARATION OF LPS FOR COMPLEXING

5.9.1.2 Pool the aliquots of bulk LPS into a clean, sterile, calibrated 5 L bottle. Measure the total volume and determine the total amount of LPS.
5.9.1.3 Add a volume of 2x TEEN buffer equal to the volume of LPS used in step 5.9.1.2, measure combined total volume, and then add a stir bar and mix with stir bar and stir plate combination for 15 ± 2 minutes.

### 5.9.2 PREPARATION OF PROTEOSOMES FOR COMPLEXING

5.9.2.1 Record the total time, if any, proteosomes was allowed to thaw.
5.9.2.2 Pool the aliquots of bulk proteosomes into a clean, sterile, 1 L graduated cylinder. Measure the total volume and determine the total amount of proteosomes.
5.9.2.3 From the cylinder in step 5.9.2.2, transfer to another clean, sterile, 1 L graduated cylinder, a volume of proteosomes containing the amount in mg equal to the mg amount of LPS used in step 5.9.1.2.
5.9.2.4 To the cylinder containing the proteosomes, add 0.22 µ filtered 30% empigen BB using 30 ml empigen per L proteosomes. Mix with a stir bar for 15 ± 2 minutes.

### 5.9.3 COMBINE PROTEOSOMES WITH THE P. SHIGELLOIDES LPS

5.9.3.1 Under gentle stirring with a stir bar, add the proteosomes from step
5.9.3.2 to the graduated 5 L bottle with the LPS and stir for 15 ± 2 minutes.
5.9.3.3 Remove four 1 ml samples and store at -75°C for later measurement of the concentration of protein by the Lowry method and LPS by KDO assay.

### 5.9.4 REMOVAL OF DETERGENT BY ULTRAFILTRATION / DIAFILTRATION

5.9.4.1 Ultrafiltration System Details System: A/G Technology, Inc. hollow fiber cartridge, 10,000 NMWC pore size, housing size 6, ultrafiltration cartridge **Note:**It is necessary to condition new cartridges to flush out glycerol. This can be done by flushing 6 liters of WFI through the 6 sq/ft cartridges or less with no back pressure. The permeate solution should not be recycled to the feed reservoir. **Note**:All ultrafiltration steps will be followed by cleaning, sanitization, and storage steps. New cartridges have to be cleaned and sanitized prior to use.
5.9.4.2 Set-up and sanitize the A/G ultrafiltration system. Clean and sanitize the system by recirculating for a minimum of 60 minutes with 2-3 liters of 0.5 N Sodium Hydroxide at an initial temperature of 50 ± 5°C. Flush the system with 4-5 liters of WFI followed by recirculating 2-3 liters of "TNS" (0.05 M Tris/normal saline buffer pH 8.0 ± 0.2, for at least 20 ± 5 minutes.
5.9.4.3 Measure the hold up volume in the UF system (including tubing) by transferring inlet and outlet lines filled with liquid out of the reservoir and into a 1 liter graduated cylinder, then pump until the UF system is empty.
5.9.4.4 Place a 2 liter side-armed vessel in a pan and pack the vessel with wet ice for the duration of the diafiltration procedure.
5.9.4.5 Transfer 1.7-1.9 L of the bulk proteosome-LPS mixture from step 5.9.3.3 into the 2 L side-armed vessel. Place a two-holed stopper fitted with two 10 ml pipettes on the vessel and connect the inlet and outlet tubing from the diafiltration system to the vessel pipettes.
5.9.4.6 Turn on the recirculation pump and adjust the pump setting to 4-6. Adjust the back pressure clamp to obtain an inlet pressure of 15 *±* 4 psi. Concentrate to a total volume of 1.0 ± 0.1 L including the hold-up volume
5.9.4.7 Transfer 1 ± 0.1 L of mixture from step 5.9.3.3 to the 2 L side-armed vessel and repeat step 5.9.4.6.
5.9.4.8 Continue transferring as in step 5.9.4.7 and concentrating as in step
5.9.4.9 until all the proteosome-LPS mixture from step 5.9.3.3 has been transferred to the 2 L side-armed vessel and the retentate volume is 1.4 ± 0.2 L which includes the hold-up volume.
5.9.4.10 Set up the ultrafiltration apparatus for continuous feed of TNS (0.05 M Tris, normal saline) into the 2 liter side-armed vessel as liquid is removed through the membrane. Set up a reservoir using a 10 liter bottle containing TNS and fitted with a tube extending to the bottom of the bottle. Connect the inlet and outlet lines from the ultrafiltration system. Turn on the recirculation pump and adjust the pump setting to 4-6. Adjust the back pressure clamp to obtain an inlet pressure of 15 ± 4 psi.
5.9.4.11 Measure the permeate flow rate on the UF unit and record the inlet pressure when the measurement is taken.
5.9.4.12 Collect a 15 to 20 ml sample of the permeate every 12 ± 0.5 liters processed, apply an in-house label ("Permeate No. P1, P2, P3 etc. ") and record the time, volume processed and maximum O.D.₆₀₀ of the sample. Verify progressive removal of empigen by testing the samples for the presence of Empigen BB using the Empigen Precipitin Test. This test consists of making serial dilutions of the permeate, and, separately, of a solution containing a known quantity of empigen BB, adding HCl to acidify the solutions and then adding serial dilutions of SDS (sodium dodecyl sulfate) to form a checkerboard-type assay. Maximum precipitate will form when equivalent amounts of empigen and SDS are present. In this manner, the amount of empigen present can be quantitated by noting the dilutions of the permeates and the SDS in which the precipitate is formed and comparing those dilutions to those of the standards. As the diafiltration progresses, the amount of empigen becomes less and the tube containing less SDS will be the tube with the maximum precipitate. The presence of precipitate is quantitated by measuring the OD at 600 nm using a spectrophotometer.
   For the amounts of components used in this example, continue diafiltration until at least 120 liters of permeate has been processed and there is a lack of significant precipitate in the Empigen precipitin test (Maximum OD 600 nm less than 0.05).
5.9.4.13 Concentrate the product to a final retentate volume of 500 ± 50 ml including the hold-up volume (step 9.4.3). Remove a 0.5 ml sample, dilute the sample 1:10, and measure the O.D.₂₈₀.
5.9.4.14 The desired minimum O.D. of the concentrated retentate is 5.7. If the O.D. is less than 5.7, continue concentrating to 400 ± 50 ml including the hold-up volume (step 5.9.4.3) and repeat the O.D.₂₈₀.
5.9.4.15 With the retentate lines out of the retentate solution and the filtrate outlet lines closed, slowly pump in the reverse direction until the cartridge and lines are empty. Transfer the feed line and retentate lines from the retentate bottle to a new clean vessel with 350 ± 50 ml TNS. Reverse the pump and slowly recirculate the TNS for 2-3 minutes.
5.9.4.16 After recirculating the TNS, stop the pump, drain the system, collect the retentate wash solution into a clean, sterile, 1 L graduated cylinder and measure the volume and the O.D.₂₈₀.
5.9.4.17 Combine the original retentate solution (step 5.9.4.12 or 5.9.4.13) with the wash retentate solution (step 5.9.4.15) and measure the final volume of retentate. Store the retentate bulk at 4°C ± 2°C until aseptic filtration.
5.9.4.18 Clean the filtration unit using WFI, followed by 0.5 N sodium hydroxide at an initial 50 ± 5°C for a minimum of 60 minutes. Rinse the cleaning agent out with of WFI then flush with 3-4 liters of 0.1 N NaOH as the storage agent.

### 5.9.5 STERILE FILTRATION

The complexes may, if desired, be sterile filtered. Obtain the bulk complexed proteosomes and LPS and inspect for the presence of any precipitate or cloudiness. If the bulk complexed product is clear, aseptically filter it using a Millipak 40, 0.22 µ filtration unit into a sterile, depyrogenated vessel. This step is to be done in a Sterile Cabinet.
**5.9.6 SPECIFIC PURPOSE:** Combine bulk GMP preparations of meningococcal outer membrane protein proteosomes and *P. shigelloides* lipopolysaccharide (for S. sonnei) into non-covalent complexes by removal of detergent.
**5.9.7 APPLICATIONS:** This GMP procedure results in the production of a bulk preparation formation of non-covalent complexes of proteosomes with *P. shigelloides 2a* lipopolysaccharide for use as a vaccine against *Shigella sonnei* infection.

### 5.10 IMMUNOGENICITY STUDIES:

### 5.10.1 IMMUNOGENICITY OF PROTEOSOME-P. SHIGELLOIDES LPS VACCINE USING HOLLOW FIBER ULTRAFILTRATION / DIAFILTRATION.

Figures 1 and 2 demonstrate that three different proteosome-shigella LPS vaccine preparations made using the hollow fiber (HF) diafiltration technique (HF-1, HF-2 and GMP/HF-3) to remove detergent and facilitate complexing are more effective at the lowest, most stringent dose tested, than preparations made using simple dialysis tubing (DT). In other words, stronger IgG (Figure 1) and IgA (Figure 2) immune responses were elicited by vaccines produced using the instant invention than by the older, less efficient technology. Since stronger immune responses result in better levels of protection, these data clearly show that the instant invention results in vaccines that are not only more efficient to make but also more powerful. Furthermore, the stronger responses are elicited by the vaccine when administered at the lowest dose (0.1 ug per dose) thereby indicating that less vaccine would be needed using the instant invention than would be required using the older technology. Since in the formulation of multivalent vaccines requires many different antigens, in order to minimize the total amount of vaccine administered, it is highly advantageous to be able to use vaccine components that are effective at lower doses. Thus, the technology of the instant invention to produce vaccines using the hollow fiber technology directly impacts and facilitates the ability to formulate multivalent vaccines having a wide range of specificities. Since it is advantageous for commercial development of vaccines to be able to lyophilize vaccines, we were gratified to discover that lyophilization of the HF vaccine complexes from a water solution, surprisingly resulted in vaccine that consistently elicited responses that were among the highest at all doses tested. Thus, higher immunogenicity for anti-LPS IgG (Figure 1) and IgA (Figure 2) was found when 0.1 ug of vaccine made by the HF technique (with or without lyophilization) was administered. These data are significant since the scale-up and GMP procedure of the instant invention used the HF technology as indicated in Figures 1 and 2 by the cross-hatched bar representing GMP/HF-3.

### 5.10.2 IMMUNOGENICITY STUDIES USING MULTIVALENT VACCINE

As shown in Figure 3, Administration of the proteosome-*P. shigelloides* LPS (for *S. sonnei* shigellosis) and the proteosome-S. *flexneri 2a* vaccines on the same day or weeks apart results in good immunogenicity to both LPS components: *S. sonnei* LPS and *S. flexneri 2a* LPS.

### 5.11 ELECTRON MICROGRAPH OF PROTEOSOME-SHIGELLA VACCINE

The association of shigella LPS with proteosomes is clearly and dramatically depicted in the electron micrograph shown in Figure 4. In this Figure, the proteosome vesicles are studded with radio-opaque black dots. These dots are gold beads linked to secondary antibodies that recognize specific anti-shigella LPS antibodies. Thus, the presence of gold dots indicates the presence of shigella LPS. Since, as can be seen, the gold dots surround and dot the proteosome vesicles, the image of proteosome vaccines with LPS non-covalently linked to the proteosomes is thereby confirmed and visualized. Surprisingly, the consistent vesicular nature of the vaccines made with the HF technology of the instant invention was not found when the DT preparations using the older technology were examined by electron microscopy (unpublished results).

### 5.12 PROTEOSOME-P. SHIGELLOIDES LPS VACCINE PROTECTS AGAINST LETHAL SHIGELLA SONNEI PNEUMONIA IN AN ANIMAL MODEL OF SHIGELLOSIS

As can be seen in Figure 5, the proteosome-*P. shigelloides* LPS vaccine consistently confers highly significant protection against lethal infection with *P. shigelloides* as measured in an animal model of shigellosis in which the animals are challenged with live organisms to induce lethal pneumonia. These data also demonstrate that not only are the correct and protective antibodies produced by the proteosome-*P. shigelloides* LPS vaccine, but also that this intranasal sub-unit vaccine can protect against lethal pneumonia.

### 5.13 INTRANASAL OR ORAL IMMUNIZATION WITH PROTEOSOME-SHIGELLA flexneri 2a LPS VACCINES INDUCE ANTI-SHIGELLA LPS IgG AND IgA IN SERUM AND IgA IN INTESTINAL AND LUNG LAVAGE FLUIDS THAT CAN LAST 30-60 DAYS POST-IMMUNIZATION.

As shown in Figure 6, two immunizations with proteosome-shigella flexneri 2a LPS vaccines induce antibodies in sera and in lung and intestinal secretions that can last 30 to 60 days post immunization. These data show that proteosome vaccines can stimulate the common mucosal immune system to secrete specific antibodies even at locations far away from the immunizing site. Specifically, intranasal immunization can induce antibodies in intestinal secretions and vice versa. This ability expands the potential utility of proteosome vaccines to protect against pathogens that invade the host through the mucosal portals of entry throughout the body.
5.14 Figure 7 shows the induction of anti-shigella LPS IgA, IgG and IgM peripheral blood ASC responses, and serum, salivary and urinary IgA and IgG antibody responses after intranasal or oral immunization of human volunteers with one or two doses proteosome-*P. shigelloides* LPS vaccines for *Shigella sonnei* using different vaccine amounts. As shown, intranasal immunization elicited responses in a dose dependant fashion with the highest dose inducing responses in all six volunteers.
   IgA, IgG and IgM serum responses were elicited in each of the intranasal groups. In addition, strong antibody secreting cell ASC responses were induced indicating that mucosal immunization stimulated trafficking of antibody secreting cells - most notably, IgA-secreting cells. Most importantly, IgA responses were also found in salivary and, especially, urinary samples indicating that secretory IgA was produced at mucosal surfaces (since IgA does not pass through the kidney, the urinary IgA reflects local secretion of antibody and suggests that such intranasal vaccines also produce local intestinal IgA and that intranasal vaccines could be used to protect against urinary tract infections caused by gram negative organisms.) It is noteworthy that the majority of these ASC and antibody responses were found after only one immunization suggesting that booster immunization may not be necessary. IgA and IgG ASCs and urinary IgA were also found after oral immunization. The most effective use of oral administration may be as a booster immunization after nasal priming, if necessary.
5.15 Demonstration of non-covalent complexing of the multimolecular proteosome proteins and LPS is shown in Figure 8. The graph shows LPS in HPLC fractions after applying uncomplexed *P. shigelloides* LPS which occurs at a different part of the column elution profile than when applying the proteosome-LPS vaccine. The co-elution of LPS and proteins in HPLC fractions after applying proteosome-*P. shigelloides* LPS vaccine is shown with peaks that correspond to the largest protein aggregates of proteosomes. The data were measured using an inhibition ELISA to quantitate the LPS and using the OD at A280 to quantitate the proteosome proteins. The HPLC column was a Tosohaas G50000Pwxl and the molecular weight standards are indicated by the arrows.

## Claims

1. A process for the preparation of proteosome-amphiphiiic determinant vaccine comprising:
(a) forming a mixture of proteosome, a dialysable detergent and an amphiphilic determinant;
(b) subjecting the mixture to diafiltration or ultrafiltration to remove the detergent thereby forming an amphiphilic determinant-proteosome complex;
(c) monitoring the amount of amphiphilic determinant-proteosome complex formation; and
(d) recovering the amphiphilic determinant-proteosome complex, wherein the nominal molecular weight cut off of the system is 3,000 or greater.

2. A process for the preparation of proteosome-amphiphilic determinant vaccine according to claim 1 wherein the ultrafiltration involves tangential flow.

3. A process for the preparation of proteosome-amphiphilic determinant vaccine according to claim 1 wherein the ultrafiltration is conducted in a system selected from hollow fiber cartridge, platform membrane and membrane cartridge.

4. A process for the preparation of proteosome-amghiphilic determinant vaccine according to claim 3 wherein the ultrafiltration system is a hollow fiber cartridge with a nominal molecular weight cut off pore size of 10,000.

5. A process for the preparation of proteosome-amphiphilic determinant vaccine according to claim 3 wherein the ultrafiltration system is a hollow fiber cartridge with a nominal molecular weight cut off pore size of 3,000.

6. A process for the preparation of proteosome-amphiphilic determinant vaccine according to claim 1, further comprising monitoring of the dialyzable detergent by continual measuring of permeate samples.

7. A process for the preparation of proteosome-amphiphilic determinant vaccine according to claim 1 wherein monitoring includes measuring the optical density of permeate or retentate sample.

8. A process for the preparation of proteosome-amphiphilic determinant vaccine according to claim 1 wherein the monitoring involves measuring the amount of detergent removal by mixing a permeate sample with a known amount of SDS (sodium dodecyl sulfate) to form a precipitate if detergent is present, and determining the amount of precipitation.

9. A process for the preparation of proteosome-amphiphilic determinant vaccine according to claim 8 wherein the proteosome-amphiphilic determinant complex is recovered when the detergent in the permeate has been essentially removed.

10. A process for the preparation of proteosome-amphiphilic determinant vaccine according to claim 1 wherein the detergent is Empigen BB.

11. A process for the preparation of proteosome-amphiphilic determinant vaccine according to claim 1 wherein the proteosome is derived from *N. meningitidis* or *N. gonorrhea*.

12. A process for the preparation of proteosome-amphiphilic determinant vaccine according to claim 1 wherein the recovery of the proteosome-amphiphilic determinant is effected when the monitored rate of detergent removal is decreasing or constant.

13. A process for the preparation of proteosome-amphiphilic determinant vaccine according to claim 1 further comprising e) mixing the recovered proteosome-amphiphilic determinant complex with a physiologically acceptable carrier to form a vaccine.

14. A process for the preparation of proteosome-amphiphilic determinant vaccine according to claim 13 further comprising concentrating the proteosome-amphiphilic determinant complex prior to recovery of the complex.

15. A process for the preparation of proteosome-amphiphilic determinant vaccine according to claim 14 wherein the concentration is continued to a desired final concentration.

16. A process for the preparation of proteosome-amphiphilic determinant vaccine according to claim 13 wherein the process is performed with different amphiphilic determinants types to form a series of proteosome-amphiphilic determinant complexes that are assembled in step (e) to form a multivalent vaccine.

17. A process for the preparation of proteosome-amphiphilic determinant vaccine according to claim 1 wherein the amphiphilic determinant is a lipopolysaccharide.

18. A process for the preparation of proteosome-lipopolysaccharide vaccine according to claim 17 wherein the lipopolysaccharide is obtained from a gram negative bacteria.

19. A process for the preparation of proteosome-lipopolysaccharide vaccine according to claim 18 wherein the gram negative bacteria is a *plesiomonas*.

20. A process for the preparation of proteosome-lipopolysaccharide vaccine according to claim 19 wherein the *plesiomonas* is *Plesiomonas shigelloides*.

21. A process for the preparation of proteosome-lipopolysaccharide vaccine according to claim 18 wherein the gram negative bacteria is a *shigella*.

22. A process for the preparation of proteosome-lipopolysaccharide vaccine according to claim 21 wherein the *shigella* is *Shigella flexneri, Shigella sonnei, or Shigella boydii*.

23. A process for the preparation of proteosome-lipopolysaccharide vaccine according to claim 18 wherein the gram negative bacteria is a *neisseria*.

24. A process for the preparation of proteosome-lipopolysaccharide vaccine according to claim 23 wherein the *neisseria* is *Neisseria meningitidis*.

25. A process for the preparation of proteosome-amphiphilic determinant vaccine according to claim 23 or 24 wherein the proteosome is derived from *N. meningitidis* or *N. gonorrhea*.

26. A process for the preparation of proteosome-amphiphilic determinant vaccine according to claim 17 further comprising e) mixing the recovered proteosome-amphiphilic determinant complex with a physiologically acceptable carrier to form a vaccine.

27. A process for the preparation of proteosome-amphiphilic determinant vaccine according to claim 26 wherein the process is performed with different amphiphilic determinants types to form a series of proteosome-amphiphilic determinant complexes that are assembled in step (e) to form a multivalent vaccine.

28. A multivalent vaccine prepared by the process of claim 26 or 27 for use in a method of treatment of the human or animal body for the prevention of disease.

## Patentansprüche

1. Ein Verfahren zur Herstellung eines Proteosom-amphiphile Determinante-Impfstoffs, wobei das Verfahren
(a) das Bilden eines Gemischs aus Proteosom, einem dialysierbaren Detergens und einer amphiphilen Determinante,
(b) das Diafiltrieren oder Ultrafiltrieren des Gemischs zur Entfernung des Detergens, wodurch ein amphiphile Determinante-Proteosom-Komplex gebildet wird,
(c) das Überwachen des Ausmaßes der Bildung des amphiphile Determinante-Proteosom-Komplexes, und
(d) das Gewinnen des amphiphile Determinante-Proteosom-Komplexes umfasst, wobei die Nenn-Molekulargewichtsgrenze des Systems 3000 oder größer ist.

2. Verfahren zur Herstellung eines Proteosom-amphiphile Determinante-Impfstoffs nach Anspruch 1, bei dem die Ultrafiltration eine tangentiale Strömung umfasst.

3. Verfahren zur Herstellung eines Proteosom-amphiphile Determinante-Impfstoffs nach Anspruch 1, bei dem die Ultrafiltration in einem System durchgeführt wird, das aus einer Hohlfaserkartusche, einer Plattformmembran und einer Membrankartusche ausgewählt ist.

4. Verfahren zur Herstellung eines Proteosom-amphiphile Determinante-Impfstoffs nach Anspruch 3, bei dem das Ultrafiltrationssystem eine Hohlfaserkartusche mit einer Nenn-Molekulargewichtsgrenze-Porengröße von 10000 ist.

5. Verfahren zur Herstellung eines Proteosom-amphiphile Determinante-Impfstoffs nach Anspruch 3, bei dem das Ultrafiltrationssystem eine Hohlfaserkartusche mit einer Nenn-Molekulargewichtsgrenze-Porengröße von 3000 ist.

6. Verfahren zur Herstellung eines Proteosom-amphiphile Determinante-Impfstoffs nach Anspruch 1, das ferner das Überwachen des dialysierbaren Detergens durch kontinuierliches Messen von durchgedrungenen Proben umfasst.

7. Verfahren zur Herstellung eines Proteosom-amphiphile Determinante-Impfstoffs nach Anspruch 1, bei dem das Überwachen die Messung der optischen Dichte der durchgedrungenen oder der zurückgehaltenen Probe umfasst.

8. Verfahren zur Herstellung eines Proteosom-amphiphile Determinante-Impfstoffs nach Anspruch 1, bei dem das Überwachen die Messung des Ausmaßes der Detergensentfemung durch Mischen einer durchgedrungenen Probe mit einer bekannten SDS-Menge (Natriumdodecylsulfat-Menge), wobei ein Niederschlag gebildet wird, wenn Detergens vorhanden ist, und die Bestimmung der Menge des Niederschlags umfasst.

9. Verfahren zur Herstellung eines Proteosom-amphiphile Determinante-Impfstoffs nach Anspruch 8, bei dem der Proteosom-amphiphile Determinante-Komplex gewonnen wird, wenn das Detergens in der durchgedrungenen Flüssigkeit im Wesentlichen entfernt worden ist.

10. Verfahren zur Herstellung eines Proteosom-amphiphile Determinante-Impfstoffs nach Anspruch 1, bei dem das Detergens Empigen BB ist.

11. Verfahren zur Herstellung eines Proteosom-amphiphile Determinante-Impfstoffs nach Anspruch 1, bei dem das Proteosom von N. meningitidis oder N. gonorrhea abgeleitet ist.

12. Verfahren zur Herstellung eines Proteosom-amphiphile Determinante-Impfstoffs nach Anspruch 1, bei dem die Gewinnung von Proteosom-amphiphile Determinante bewirkt wird, wenn die überwachte Geschwindigkeit der Detergensentfernung abnimmt oder konstant ist.

13. Verfahren zur Herstellung eines Proteosom-amphiphile Determinante-Impfstoffs nach Anspruch 1, das ferner (e) das Mischen des gewonnenen Proteosom-amphiphile Determinante-Komplexes mit einem physiologisch verträglichen Träger zur Bildung eines Impfstoffs umfasst.

14. Verfahren zur Herstellung eines Proteosom-amphiphile Determinante-Impfstoffs nach Anspruch 13, das ferner die Konzentrierung des Proteosom-amphiphile Determinante-Komplexes vor der Gewinnung des Komplexes umfasst.

15. Verfahren zur Herstellung eines Proteosom-amphiphile Determinante-Impfstoffs nach Anspruch 14, bei dem die Konzentrierung bis zu einer gewünschten Endkonzentration fortgesetzt wird.

16. Verfahren zur Herstellung eines Proteosom-amphiphile Determinante-Impfstoffs nach Anspruch 13, wobei das Verfahren mit verschiedenen amphiphilen Determinantentypen zur Bildung einer Reihe von Proteosom-amphiphile Determinante-Komplexen, die im Schritt (e) zusammengesetzt werden, zur Bildung eines multivalenten Impfstoffs durchgeführt wird.

17. Verfahren zur Herstellung eines Proteosom-amphiphile Determinante-Impfstoffs nach Anspruch 1, bei dem die amphiphile Determinante ein Lipopolysaccharid ist.

18. Verfahren zur Herstellung eines Proteosom-Lipopolysaccharid-Impfstoffs nach Anspruch 17, bei dem das Lipopolysaccharid von einem gramnegativen Bakterium erhalten worden ist.

19. Verfahren zur Herstellung eines Proteosom-Lipopolysaccharid-Impfstoffs nach Anspruch 18, bei dem das gramnegative Bakterium ein Plesiomonas ist.

20. Verfahren zur Herstellung eines Proteosom-Lipopolysaccharid-Impfstoffs nach Anspruch 19, bei dem das Plesiomonas Plesiomonas shigelloides ist.

21. Verfahren zur Herstellung eines Proteosom-Lipopolysaccharid-Impfstoffs nach Anspruch 18, bei dem das gramnegative Bakterium ein Shigella ist,

22. Verfahren zur Herstellung eines Proteosom-Lipopolysaccharid-Impfstoffs nach Anspruch 21, bei dem das Shigella Shigella flexneri, Shigella sonnei oder Shigella boydii ist,

23. Verfahren zur Herstellung eines Proteosom-Lipopolysaccharid-Impfstoffs nach Anspruch 18, bei dem das gramnegative Bakterium ein Neisseria ist.

24. Verfahren zur Herstellung eines Proteosom-Lipopolysaccharid-Impfstoffs nach Anspruch 23, bei dem das Neisseria Neisseria meningitidis ist.

25. Verfahren zur Herstellung eines Proteosom-amphiphile Determinante-Impfstoffs nach Anspruch 23 oder 24, bei dem das Proteosom von N. meningitidis oder N, gonorrhea abgeleitet ist.

26. Verfahren zur Herstellung eines Proteosom-amphiphile Determinante-Impfstoffs nach Anspruch 17, das ferner (e) das Mischen des gewonnenen Proteosom-amphiphile Determinante-Komplexes mit einem physiologisch verträglichen Träger zur Bildung eines Impfstoffs umfasst.

27. Verfahren zur Herstellung eines Proteosom-amphiphile Determinante-Impfstoffs nach Anspruch 26, wobei das Verfahren mit verschiedenen amphiphilen Determinantentypen zur Bildung einer Reihe von Proteosom-amphiphile Determinante-Komplexen, die im Schritt (e) zusammengesetzt werden, zur Bildung eines multivalenten Impfstoffs durchgeführt wird.

28. Ein multivalenter Impfstoff, der mit dem Verfahren nach Anspruche 26 oder 27 hergestellt wird, zur Verwendung in einem Verfahren zur Behandlung des Menschen- oder Tierkörpers zur Verhinderung einer Erkrankung.

## Revendications

1. Procédé pour la préparation d'un vaccin à base de protéosome-déterminant amphiphile, comportant les étapes consistant à :
(a) former un mélange de protéosome, d'un détergent dialysable et d'un déterminant amphiphile ;
(b) soumettre le mélange à une diafiltration ou ultrafiltration pour éliminer le détergent de manière à former un complexe déterminant amphiphile-protéosome ;
(c) surveiller la quantité de formation du complexe déterminant amphiphile-protéosome ; et
(d) récupérer le complexe déterminant amphiphile-protéosome, dans lequel la coupure de poids moléculaire nominal du système est de 3000 ou plus.

2. Procédé pour la préparation d'un vaccin à base de protéosome-déterminant amphiphile selon la revendication 1, dans lequel l'ultrafiltration implique un écoulement tangentiel.

3. Procédé pour la préparation d'un vaccin à base de protéosome-déterminant amphiphile selon la revendication 1, dans lequel l'ultrafiltration est effectuée dans un système sélectionné parmi une cartouche à fibre creuse, une membrane plate-forme et une cartouche à membrane.

4. Procédé pour la préparation d'un vaccin à base de protéosome-déterminant amphiphile selon la revendication 3, dans lequel le système d'ultrafiltration est une cartouche à fibre creuse ayant une taille de pore à coupure de poids moléculaire nominal de 10000.

5. Procédé pour la préparation d'un vaccin à base de protéosome-déterminant amphiphile selon la revendication 3, dans lequel le système d'ultrafiltration est une cartouche à fibre creuse ayant une taille de pore à coupure de poids moléculaire nominal de 3000.

6. Procédé pour la préparation d'un vaccin à base de protéosome-déterminant amphiphile selon la revendication 1, comportant de plus la surveillance du détergent dialysable par une mesure continue d'échantillons de perméat.

7. Procédé pour la préparation d'un vaccin à base de protéosome-déterminant amphiphile selon la revendication 1, dans lequel la surveillance inclut la mesure de la densité optique de l'échantillon de perméat ou de rétentat.

8. Procédé pour la préparation d'un vaccin à base de protéosome-déterminant amphiphile selon la revendication 1, dans lequel la surveillance implique la mesure de la quantité d'élimination de détergent en mélangeant un échantillon de perméat avec une quantité connue de SDS (dodécylsulfate de sodium) pour former un précipité si le détergent est présent, et la détermination de la quantité de précipitation.

9. Procédé pour la préparation d'un vaccin à base de protéosome-déterminant amphiphile selon la revendication 8, dans lequel le complexe protéosome-déterminant amphiphile est récupéré lorsque le détergent dans le perméat a été essentiellement éliminé.

10. Procédé pour la préparation d'un vaccin à base de protéosome-déterminant amphiphile selon la revendication 1, dans lequel le détergent est Empigen BB.

11. Procédé pour la préparation d'un vaccin à base de protéosome-déterminant amphiphile selon la revendication 1, dans lequel le protéosome est dérivé de *N. meningitidis* ou *N. gonorrhoeae*.

12. Procédé pour la préparation d'un vaccin à base de protéosome-déterminant amphiphile selon la revendication 1, dans lequel la récupération du complexe protéosome-déterminant amphiphile est effectuée lorsque le taux surveillé d'élimination du détergent diminue ou est constant.

13. Procédé pour la préparation d'un vaccin à base de protéosome-déterminant amphiphile selon la revendication 1, comportant de plus e) le mélange du complexe protéosome-déterminant amphiphile récupéré avec un support physiologiquement acceptable pour former un vaccin.

14. Procédé pour la préparation d'un vaccin à base de protéosome-déterminant amphiphile selon la revendication 13, comportant de plus la concentration du complexe protéosome-déterminant amphiphile avant la récupération du complexe.

15. Procédé pour la préparation d'un vaccin à base de protéosome-déterminant amphiphile selon la revendication 14, dans lequel la concentration est poursuivie jusqu'à une concentration finale voulue.

16. Procédé pour la préparation d'un vaccin à base de protéosome-déterminant amphiphile selon la revendication 13, dans lequel le procédé est effectué avec des types de déterminants amphiphiles différents pour former une série de complexes protéosome-déterminant amphiphile qui sont assemblés à l'étape (e) pour former un vaccin multivalent.

17. Procédé pour la préparation d'un vaccin à base de protéosome-déterminant amphiphile selon la revendication 1, dans lequel le déterminant amphiphile est un lipopolysaccharide.

18. Procédé pour la préparation d'un vaccin à base de protéosome-lipopolysaccharide selon la revendication 17, dans lequel le lipopolysaccharide est obtenu à partir d'une bactérie à gram négatif.

19. Procédé pour la préparation d'un vaccin à base de protéosome-lipopolysaccharide selon la revendication 18, dans lequel la bactérie à gram négatif est *plesiomonas*.

20. Procédé pour la préparation d'un vaccin à base de protéosome-lipopolysaccharide selon la revendication 19, dans lequel la bactérie *plesiomonas* est *Plesiomonas shigelloides*.

21. Procédé pour la préparation d'un vaccin à base de protéosome-lipopolysaccharide selon la revendication 18, dans lequel la bactérie à gram négatif est *shigella*.

22. Procédé pour la préparation d'un vaccin à base de protéosome-lipopolysaccharide selon la revendication 21, dans lequel la bactérie *shigella* est *Shigella* flexneri, *Shigella sonnei*, ou *Shigella boydii*.

23. Procédé pour la préparation d'un vaccin à base de protéosome-lipopolysaccharide selon la revendication 18, dans lequel la bactérie à gram négatif est *neisseria*.

24. Procédé pour la préparation d'un vaccin à base de protéosome-lipopolysaccharide selon la revendication 23, dans lequel la bactérie *neisseria* est *Neisseria meningitidis*.

25. Procédé pour la préparation d'un vaccin à base de protéosome-déterminant amphiphile selon la revendication 23 ou 24, dans lequel le protéosome est dérivé de *N. meningitidis* ou *N. gonorrhoeae*.

26. Procédé pour la préparation d'un vaccin à base de protéosome-déterminant amphiphile selon la revendication 17, comportant de plus e) le mélange du complexe protéosome-déterminant amphiphile récupéré avec un support physiologiquement acceptable pour former un vaccin.

27. Procédé pour la préparation d'un vaccin à base de protéosome-déterminant amphiphile selon la revendication 26, dans lequel le procédé est effectué avec des types de déterminants amphiphiles différents pour former une série de complexes protéosome-déterminant amphiphile qui sont assemblés à l'étape (e) pour former un vaccin multivalent.

28. Vaccin multivalent préparé par le procédé selon la revendication 26 ou 27, destiné à être utilisé dans un procédé de traitement du corps humain ou animal pour la prévention d'une maladie.
